(19) **Europäisches Patentamt / European Patent Office / Office européen des brevets**

(11) **EP 3 465 153 B1**

(12) # FASCICULE DE BREVET EUROPEEN

(45) Date de publication et mention
de la délivrance du brevet:
**06.09.2023 Bulletin 2023/36**

(21) Numéro de dépôt: **17721588.6**

(22) Date de dépôt: **27.04.2017**

(51) Classification Internationale des Brevets (IPC):
**G01N 21/45** *(2006.01)*     **G03H 1/00** *(2006.01)*
**G03H 1/08** *(2006.01)*

(52) Classification Coopérative des Brevets (CPC):
**G03H 1/0866; G01N 15/1468; G03H 1/041;**
**G03H 1/0443;** G01N 21/453; G01N 2015/1006;
G01N 2015/1454; G03H 2001/0447;
G03H 2001/0452

(86) Numéro de dépôt international:
**PCT/EP2017/060027**

(87) Numéro de publication internationale:
**WO 2017/207184 (07.12.2017 Gazette 2017/49)**

(54) **DISPOSITIF ET PROCEDE D'ACQUISITION D'UNE PARTICULE PRESENTE DANS UN ECHANTILLON**

VORRICHTUNG UND VERFAHREN ZUR ERFASSUNG EINES PARTIKELS, DAS IN EINER PROBE ENTHALTEN IST

DEVICE AND METHOD FOR ACQUIRING A PARTICLE PRESENT IN A SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.05.2016 EP 16305625**

(43) Date de publication de la demande:
**10.04.2019 Bulletin 2019/15**

(73) Titulaires:
• **Biomérieux**
**69280 Marcy-L'Etoile (FR)**
• **Bioaster**
**69007 Lyon (FR)**

(72) Inventeurs:
• **DOUET, Alice**
**16320 Villebois-Lavalette (FR)**
• **JOSSO, Quentin**
**69007 Lyon (FR)**

(74) Mandataire: **Cabinet Laurent & Charras**
**Le Contemporain**
**50 Chemin de la Bruyère**
**69574 Dardilly Cedex (FR)**

(56) Documents cités:
**WO-A1-2016/075279**

• **JACOB P FUGAL ET AL: "Practical methods for automated reconstruction and characterization of particles in digital in-line holograms; Practical methods for automated digital particle hologram analysis", MEASUREMENT SCIENCE AND TECHNOLOGY, IOP, BRISTOL, GB, vol. 20, no. 7, 1 juillet 2009 (2009-07-01), page 75501, XP020160483, ISSN: 0957-0233**
• **AHMED EL MALLAHI ET AL: "Automated three-dimensional detection and classification of living organisms using digital holographic microscopy with partial spatial coherent source: application to the monitoring of drinking water resources", APPLIED OPTICS, OPTICAL SOCIETY OF AMERICA, WASHINGTON, DC, US, vol. 52, no. 1, 1 January 2013 (2013-01-01), pages A68-A80, XP001580209, ISSN: 0003-6935, DOI: HTTP://DX.DOI.ORG/10.1364/AO.52.000A68**

**(Cont. page suivante)**

- DUBOIS F ET AL: "FOCUS PLANE DETECTION CRITERIA IN DIGITAL HOLOGRAPHY MICROSCOPY BY AMPLITUDE ANALYSIS", OPTICS EXPRESS, OSA PUBLISHING, US, vol. 14, no. 13, 26 June 2006 (2006-06-26) , pages 5895-5908, XP007900778, ISSN: 1094-4087, DOI: 10.1364/OE.14.005895

**Description**

DOMAINE TECHNIQUE

La présente invention concerne le domaine de l'acquisition optique de particules biologiques. Les particules biologiques peuvent être des microorganismes tels que des bactéries, des champignons ou des levures par exemple. Il peut également s'agir de cellules, organismes multicellulaires, ou toute autre particule de type particule polluante, poussière.

L'invention trouve une application particulièrement avantageuse pour analyser l'état d'une particule biologique, par exemple pour savoir l'état métabolique d'une bactérie suite à l'application d'un antibiotique. L'invention permet, par exemple, de réaliser un antibiogramme d'une bactérie.

ART ANTERIEUR

Un antibiogramme est une technique de laboratoire visant à tester le phénotype d'une souche bactérienne vis-à-vis d'un ou plusieurs antibiotiques. Un antibiogramme est classiquement réalisé par culture d'un échantillon contenant des bactéries et un antibiotique.

La demande de brevet européen N° 2 603 601 décrit une méthode pour réaliser un antibiogramme en visualisant l'état des bactéries après une durée d'incubation en présence d'un antibiotique. Pour visualiser les bactéries, les bactéries sont marquées par des marqueurs fluorescents permettant de révéler leurs structures. La mesure de la fluorescence des marqueurs permet alors de déterminer si l'antibiotique a agi efficacement sur les bactéries.

Cependant, le processus de marquage est particulièrement long et complexe à réaliser et ces marqueurs chimiques ont un effet cytotoxique sur les bactéries. Il s'ensuit que ce mode de visualisation ne permet pas d'observer les bactéries à plusieurs instants de la culture des bactéries.

Il est donc nécessaire d'utiliser un temps de culture suffisamment long, de l'ordre de 24 à 72 heures, pour garantir la fiabilité de la mesure.

D'autres méthodes de visualisation de particules biologiques utilisent un microscope, permettant une mesure non destructive d'un échantillon.

La microscopie holographique numérique ou DHM (Digital Holographic Microscopy) est une technique d'imagerie permettant de s'affranchir des contraintes de profondeur de champ de la microscopie optique classique. Schématiquement, elle consiste à enregistrer un hologramme formé par l'interférence entre les ondes lumineuses diffractées par l'objet observé et une onde de référence présentant une cohérence spatiale. Cette technique est décrite dans l'article de revue de Myung K.Kim intitulé « Principles and techniques of digital holographie microscopy » publié dans SPIE Reviews Vol. 1, N°1, Janvier 2010.

Récemment, il a été proposé d'utiliser la microscopie holographique numérique pour identifier des microorganismes de manière automatisée. Ainsi, l'article de N. Wu et al. intitulé « Three-dimensional identification of microorganisms using a digital holographie microscope» publié dans Computational and Mathematical Methods in Medicine, Vol. 2013, art. N° ID 162105, décrit une méthode d'identification de différents types de bactéries dans le volume à analyser grâce à une propagation numérique vers le plan de mise au point de la particule. Les images mises au point à différentes profondeurs sont utilisées pour reconstituer une représentation tridimensionnelle des microorganismes. Ceux-ci sont alors classifiés au moyen d'un filtrage 3D non linéaire.

De manière similaire, l'article de Ahmed El Mallahi intitulé « Automated threedimensional détection and classification of living organisms using digital holography microscopy with partial spatial cohérent source: application to monitoring of drinking water resources » publié dans Applied Optics, Vol. 52 N° 1, Janvier 2013, décrit une méthode comprenant une première étape de détection de la position des bactéries dans le volume à analyser, une étape de focalisation à différentes profondeurs du volume au moyen d'une propagation numérique, puis une classification des bactéries à partir de leurs caractéristiques morphologiques.

Les méthodes d'identification précitées sont toutefois complexes dans la mesure où elles nécessitent une focalisation dans des plans successifs de mise au point.

A contrario, la focalisation dans un seul plan de mise au point, autrement dit à une seule profondeur d'analyse, ne suffit généralement pas à identifier un type de microorganisme avec un faible taux de fausse détection.

Le problème technique objectif de la présente invention est, par conséquent, d'observer une particule biologique en limitant le temps d'acquisition, c'est-à-dire sans marquage et sans une mise au point précise du système optique.

EXPOSE DE L'INVENTION

Pour résoudre le problème technique, l'invention propose un dispositif de détermination de l'état d'au moins une particule par acquisition au cours du temps d'une pluralité de particules présentes dans un échantillon et intégrant une simple acquisition sans focalisation associée à une reconstruction numérique de la focalisation comportant : une première focalisation moyenne destinée à détecter au moins une particule et une seconde focalisation spécifique d'une région d'intérêt contenant la particule.

A cet effet, un premier aspect de l'invention est défini selon le dispositif de la revendication 1.

L'invention permet ainsi d'observer des phénomènes similaires à ceux décrits dans l'état de l'art sans marquage chimique. La mise au point est effectuée de manière numérique à partir d'une image défocalisée associée à une reconstruction numérique de la focalisation comportant : une première focalisation moyenne destinée à détecter au moins une particule et une seconde focalisation spécifique d'une région d'intérêt contenant la particule.

Il s'ensuit que les instruments de mesure sont simplifiés car il n'est pas nécessaire d'utiliser des appareils de mise au point extrêmement précis permettant de mettre au point une image de quelques nanomètres. Le temps d'acquisition est également réduit car la mise au point ou le marquage n'est plus nécessaire.

En outre, les opérations de marquage et de mise au point sont classiquement réalisées manuellement. L'invention permet également de limiter ces interactions manuelles durant l'acquisition et donc d'automatiser le processus d'acquisition d'une particule. Il s'ensuit que le dispositif d'acquisition d'une particule présente dans un échantillon peut être positionné au plus près d'un patient de sorte à améliorer la rapidité de traitement d'un patient.

Le processus classique pour déterminer les antibiotiques efficaces sur une souche bactérienne consiste à réaliser un prélèvent contenant ladite souche (e.g. sur un patient, un animal, un lot alimentaire,...) puis à transmettre le prélèvement à un centre d'analyse. Lorsque le centre d'analyse réceptionne le prélèvement, il procède tout d'abord à la culture de la souche bactérienne pour obtenir au moins une colonie de celle-ci, culture comprise entre 24 heure et 72 heures. Il prépare ensuite à partir de cette colonie plusieurs échantillons comprenant des antibiotiques différents et/ou des concentrations d'antibiotiques différentes, puis met à nouveau les échantillons à incuber. Après une nouvelle durée de culture comprise également entre 24 et 72 heures, chaque échantillon est analysé manuellement pour déterminer si l'antibiotique a agi efficacement. Les résultats sont alors retransmis au praticien pour appliquer l'antibiotique et/ou la concentration d'antibiotique le plus efficace. L'invention permet de refondre entièrement ce processus en déplaçant le dispositif d'analyse proche du praticien car la manipulation subtile d'un opérateur n'est plus nécessaire pour effectuer la mise au point ou le marquage.

Typiquement, l'invention a permis de détecter les modifications structurelles d'une bactérie en présence d'un antibiotique après une incubation de seulement une dizaine de minute, et sa sensibilité au bout de deux heures (détection de la présence ou de l'absence d'une division ou d'un motif codant la division) contrairement au processus précédemment décrit qui peut prendre plusieurs jours. En effet, les mesures étant non destructives, il est possible de réaliser des analyses très tôt dans le processus de culture sans risquer de détruire l'échantillon et donc de prolonger le temps d'analyse.

Selon un mode de réalisation, ledit capteur d'image est configuré pour acquérir un flux d'images, et l'unité de traitement est configurée pour suivre une particule dans le flux de premières matrices électromagnétiques.

Ce mode de réalisation permet de suivre une particule sur plusieurs images successives de sorte à former un film représentant l'évolution d'une particule au cours du temps. La méthode classique du marquage chimique ne permet pas de représenter l'évolution d'une particule au cours du temps car les particules sont altérées après la première analyse.

La méthode classique de visualisation de particules biologiques utilisant la microscopie classique (non holographique) permet de représenter une particule au cours du temps mais elle nécessite une focalisation manuelle pour chaque image. En effet, une particule n'est pas fixe dans un échantillon et elle peut se déplacer dans le plan du support ou dans la profondeur de l'échantillon. Même si une particule se déplace uniquement dans le plan de son support, la focalisation de la particule peut être défaillante entre deux images en raison d'un défaut de planéité du support.

Ce mode de réalisation permet de représenter une particule au cours du temps en recherchant une focalisation pouvant varier selon le plan du support ou dans la profondeur de l'échantillon. Avantageusement les particules sont des microorganismes, et la durée entre deux images acquises issues dudit capteur d'image est inférieure ou égale à 1 minute.

Typiquement, ce mode de réalisation de l'invention a permis d'observer la division cellulaire d'une bactérie.

Il s'ensuit que ce mode de réalisation permet d'augmenter la fiabilité et la rapidité d'analyse de l'état d'une particule. En effet, au lieu d'analyser une particule uniquement en fonction de ses caractéristiques physiologique, il est possible d'étudier une particule en fonction de son comportement. Par exemple, il est possible de constater qu'une bactérie ne se divise plus lorsqu'un antibiotique efficace est présent dans l'échantillon.

Selon un mode de réalisation, ladite série de matrices électromagnétiques est obtenue par un modèle de propagation numérique d'une lumière à travers ledit échantillon, les matrices électromagnétiques variant en modulant une distance d'un axe optique dudit modèle de propagation. Ce mode de réalisation permet de reconstruire efficacement l'image de la particule.

Avantageusement, l'unité de traitement comprend un module de transformation des matrices électromagnétiques issues du modèle de propagation par une application surjective de l'espace complexe dans l'espace réel. Les matrices obtenues permettent une visualisation directement compréhensible par un opérateur ainsi que la mise en oeuvre de traitement d'image classique sur la base par exemple de la morphologie des particules.

Selon un mode de réalisation, la première matrice électromagnétique est obtenue en représentant, à chaque coordonnée, les composantes à ladite coordonnée des matrices électromagnétiques en fonction de ladite distance dudit axe optique, et en recherchant une moyenne des maxima de toutes les représentations. Ce mode de réalisation permet de détecter efficacement la matrice focalisée moyenne.

Selon un mode de réalisation, la seconde image électromagnétique est obtenue en représentant, à chaque coordonnée de la particule identifiée, les composantes à ladite coordonnée des matrices électromagnétiques en fonction de ladite distance dudit axe optique, et en recherchant une moyenne des maxima de toutes les représentations. Ce mode de réalisation permet de détecter efficacement la matrice focalisée sur la particule.

Selon un mode de réalisation, les matrices de ladite série de matrices électromagnétiques utilisées pour déterminer ladite première matrice électromagnétique et/ou pour déterminer ladite seconde matrice électromagnétique sont sous-échantillonnées selon ladite distance. Ce mode de réalisation permet de limiter le temps de calcul nécessaire pour les focalisations.

Selon un mode de réalisation, ledit dispositif comporte plusieurs unités d'acquisition, chaque unité d'acquisition comportant un capteur d'image et des moyens de focalisation spécifiques, ledit dispositif étant configuré pour représenter une image d'une particule de chaque échantillon. Ce mode de réalisation permet de faire des comparaisons rapides entre plusieurs échantillons, par exemple pour réaliser un antibiogramme.

Selon un second aspect, l'invention a également pour objet un procédé d'analyse de l'état d'au moins une particule présente dans un échantillon tel que défini par la revendication 10.

Selon ce second aspect, l'invention permet également d'observer des phénomènes similaires à ceux décrits dans l'état de l'art sans marquage chimique.

La mise au point est effectuée de manière numérique à partir d'une image défocalisée associée à une reconstruction numérique de la focalisation. Pour finir, l'état de la particule est déterminé à partir de l'image focalisée. Il s'ensuit que les instruments de mesure sont simplifiés car il n'est pas nécessaire d'utiliser des appareils de mise au point extrêmement précis permettant de mettre au point une image de quelques nanomètres. Le temps d'acquisition est également réduit car la mise au point ou le marquage n'est plus nécessaire.

En outre, les opérations de marquage et de mise au point sont classiquement réalisées manuellement. Selon ce second aspect, l'invention permet également de limiter ces interactions manuelles durant l'acquisition et donc d'automatiser le processus d'analyse d'une particule. Il s'ensuit que le procédé d'analyse d'une particule présente dans un échantillon peut être réalisé au plus près d'un patient de sorte à améliorer la rapidité de traitement d'un patient.

Selon un mode de réalisation, l'étape d'obtention de la matrice électromagnétique à la distance de mise au point sur la particule comporte les étapes suivantes :

- détermination d'une première matrice électromagnétique à une distance de mise au point moyenne sur les particules à partir de la série de matrices électromagnétiques ;
- l'identification de la particule dans la première matrice électromagnétique et la mémorisation des coordonnées de ladite particule ; et
- la détermination de la matrice électromagnétique à la distance de mise au point sur la particule à partir des composantes de la série de matrices électromagnétique ayant les coordonnées mémorisées.

Ce mode de réalisation permet de déterminer efficacement une matrice focalisée de ladite particule au moyen d'une première focalisation moyenne destinée à détecter au moins une particule et d'une seconde focalisation spécifique sur la particule.

Selon un mode de réalisation, ladite étape de détermination d'un état de ladite particule comporte les étapes suivantes :

- rotation de la matrice électromagnétique à la distance de mise au point sur la particule de manière à aligner la particule sur un axe prédéfini, et
- calcul d'une distribution de la matrice alignée selon ledit axe prédéfini.

Ce mode de réalisation permet de s'affranchir de l'orientation et de la position de la particule. En outre, la distribution permet de catégoriser la forme de la particule.

Selon un mode de réalisation, les étapes d'acquisition d'une image holographique jusqu'à l'étape d'obtention d'une image focalisée d'une particule sont réalisées au cours du temps pour plusieurs images, l'étape de détermination d'un état de ladite particule étant réalisée en fonction d'une évolution temporelle de ladite particule.

Ce mode de réalisation permet de représenter une particule au cours du temps en recherchant une focalisation pouvant varier selon le plan du support ou dans la profondeur de l'échantillon. Avantageusement, la durée entre deux images issues dudit capteur d'image est inférieure ou égale à 1 minutes dans le cadre de microorganisme, en particulier de bactéries.

Selon un mode de réalisation, ladite étape de détermination d'un état de ladite particule comporte les étapes suivantes :

- rotation pour chaque image acquise, de la matrice électromagnétique à la distance de mise au point sur la particule de manière à aligner la particule sur un axe prédéfini ; et
- calcul, pour chaque image acquise, d'une distribution de la matrice alignée selon ledit axe prédéfini ; et
- représentation matricielle des distributions associées à chaque image en fonction du temps.

Ce mode de réalisation permet d'améliorer la catégorisation de la particule.

Selon un mode de réalisation, ladite étape de détermination d'un état de ladite particule est réalisée en fonction d'un état de division de ladite particule.

Ce mode de réalisation permet, par exemple, de détecter si une bactérie est dans un cycle de division ou si un inhibiteur bloque ce cycle de division.

Selon un mode de réalisation, ladite étape de détermination d'un état de ladite particule est réalisée en fonction d'une caractéristique morphologique de ladite particule, par exemple la longueur.

Selon un mode de réalisation, ladite étape de détermination d'un état de ladite particule est réalisée en fonction d'une caractéristique physiologique de ladite particule, par exemple le nombre de noyau.

Selon un mode de réalisation, ladite particule correspond à une bactérie et ledit échantillon intègre un antibiotique.

## DESCRIPTION SOMMAIRE DES FIGURES

La manière de réaliser l'invention ainsi que les avantages qui en découlent, ressortiront bien du mode de réalisation qui suit, donné à titre indicatif mais non limitatif, à l'appui des figures annexées dans lesquelles les figures 1 à 6 représentent :

- figure 1 : une représentation schématique en coupe d'un dispositif d'acquisition d'une particule présente dans un échantillon selon un mode de réalisation de l'invention ;
- figure 2 : une représentation schématique d'un organigramme de fonctionnement d'une unité de traitement de la figure 1 selon un premier mode de réalisation ;
- figure 3 : une représentation schématique d'un organigramme de fonctionnement du module de détermination d'une image focalisée moyenne de la figure 2 ;
- figure 4 : une représentation schématique d'un organigramme de fonctionnement du module de détermination d'une région d'intérêt de la figure 2 ;
- figure 5 : une représentation schématique d'un organigramme de fonctionnement d'une unité de traitement de la figure 1 selon un second mode de réalisation ;
- figure 6 : une représentation schématique d'un organigramme de fonctionnement du module de prédiction d'un état d'une particule de la figure 5 ;
- figure 7 : une représentation schématique d'une image d'une matrice de distributions seuillée ;
- figure 8 : une représentation schématique d'un ensemble de vignettes d'image comprenant une bactérie observée au cours du temps grâce à un procédé selon un mode de réalisation de l'invention ; et
- figure 9 : un ensemble de distribution correspondant aux vignettes de la figure 8.

## DESCRIPTION DETAILLEE DE L'INVENTION

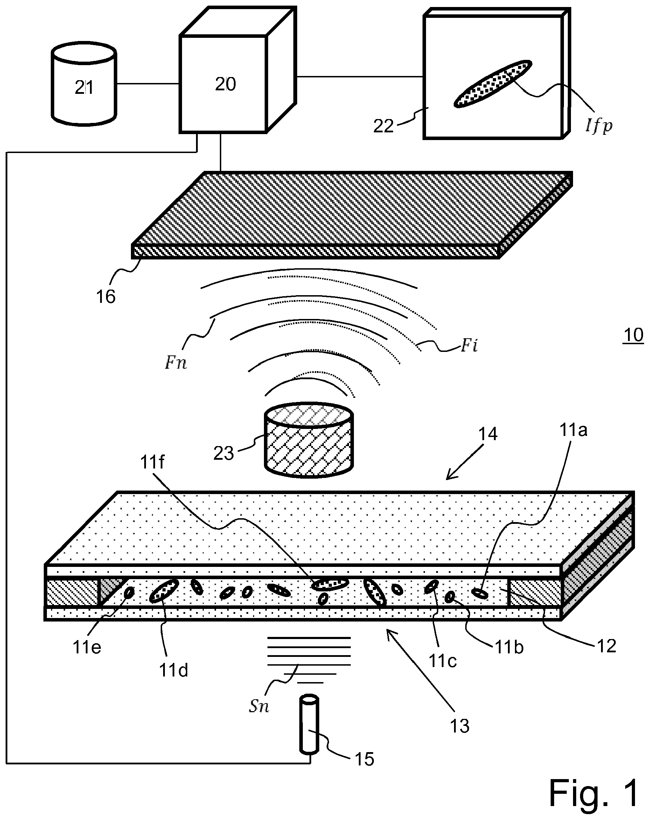

La figure 1 illustre un dispositif d'observation **10** d'une particule **11a-11f** présente dans un échantillon **12**. L'échantillon **12** est disposé entre une source lumineuse **15,** spatialement et temporellement cohérente (e.g. un laser) ou pseudo-cohérente (e.g. une diode électroluminescente, une diode laser), et un capteur numérique **16** sensible dans la gamme spectrale de la source lumineuse. De préférence, la source lumineuse **15** comporte une faible largeur spectrale, par exemple inférieure à 200nm, inférieure à 100nm ou encore inférieure à 25 nm. Dans ce qui suit, il est fait référence à la longueur d'onde d'émission centrale de la source lumineuse, par exemple dans le domaine visible. La source lumineuse **15** émet un signal cohérent *Sn* orienté sur une première face **13** de l'échantillon, par exemple acheminé par un guide d'onde tel qu'une fibre optique.

L'échantillon **12** consiste en un liquide tel que de l'eau, une solution tampon, un milieu de culture ou un milieu réactif (comprenant ou non un antibiotique), dans lequel se trouvent les particules **11a-11f** à observer.

En variante, l'échantillon **12** peut se présenter sous la forme d'un milieu solide, de préférence translucide, tel qu'une gélose agar-agar, dans lequel se trouvent les particules **11a-11f**. L'échantillon **12** peut également être un milieu gazeux. Les particules **11a-11f** peuvent se situer à l'intérieur du milieu ou bien à la surface de l'échantillon **12**.

Les particules **11a-11f** peuvent être des microorganismes tels que des bactéries, des champignons ou des levures. Il peut également s'agir de cellules, organismes multicellulaires, ou toute autre particule de type particule polluante, poussière. La taille des particules **11a-11f** observées varie entre 500nm et plusieurs centaines de $\mu$m, voire quelques millimètres.

L'échantillon **12** est contenu dans une chambre d'analyse, délimitée verticalement par une lame inférieure et une lame supérieure, par exemple des lames de microscope conventionnelles. La chambre d'analyse est délimitée latéralement par un adhésif ou par tout autre matériau étanche. Les lames inférieure et supérieure sont transparentes à la longueur d'onde de la source lumineuse **15**, l'échantillon et la chambre laissant passer par exemple plus de 50% la longueur d'onde de la source lumineuse sous incidence normale sur la lame inférieure.

De préférence, les particules **11a-11f** sont disposées dans l'échantillon **12** au niveau de la lame supérieure. La face inférieure de la lame supérieure comprend à cet effet des ligands permettant d'accrocher les particules, par exemple des polycations (e.g. poly-L-lysine) dans le cadre de microorganismes Ceci permet de contenir les particules dans une épaisseur égale à, ou proche de, la profondeur de champ du système optique, à savoir dans une épaisseur inférieure à 1mm (e.g. lentille à tube), et de préférence inférieure à 100$\mu$m (e.g. objectif de microscope). Les particules **11a-11f** peuvent néanmoins se déplacer dans l'échantillon **12**.

De préférence, le dispositif comprend un système optique **23** constitué, par exemple, d'un objectif de microscope et d'une lentille de tube, disposé dans l'air et à distance fixe de l'échantillon. Le système optique **23** est optionnellement équipé d'un filtre pouvant être situé devant l'objectif ou entre l'objectif et la lentille de tube. Le système optique **23** est caractérisé par son axe optique, son plan d'objet, également dénommé plan de mise au point, à une distance de l'objectif et son plan d'image, conjugué du plan d'objet par le système optique. En d'autres termes, à un objet situé dans le plan d'objet, correspond une image nette de cet objet dans le plan image, également appelé plan focal. Les propriétés optiques du système **23** sont fixes (e.g. optique à focale fixe). Les plans objet et image sont orthogonaux à l'axe optique.

Le capteur d'image **16** est situé, en regard d'une seconde face **14** de l'échantillon, dans le plan focal ou à proximité de ce dernier. Le capteur, par exemple un capteur CCD ou CMOS, comprend un réseau bidimensionnel périodique de sites élémentaires sensibles, et une électronique de proximité qui règle le temps d'exposition et la remise à zéro des sites, d'une manière connue en soi. Le signal de sortie d'un site élémentaire est fonction de la quantité de rayonnement de la gamme spectrale incident sur ledit site pendant la durée d'exposition. Ce signal est ensuite converti, par exemple par l'électronique de proximité, en point image, ou « pixel », d'une image numérique. Le capteur produit ainsi une image numérique sous forme d'une matrice à **C** colonnes et **L** lignes. Chaque pixel de cette matrice, de coordonnées $(c, l)$ dans la matrice, correspond d'une manière connue en soi à une position de coordonnées cartésiennes $(x(c, l), y(c,l))$ dans le plan focal du système optique **23,** par exemple la position du centre du site sensible élémentaire de forme rectangulaire.

Le pas et le facteur de remplissage du réseau périodique sont choisis pour respecter le critère Shannon-Nyquist vis-à-vis de la taille des particules observées, de manière à définir au moins deux pixels par particule. Ainsi, le capteur d'image **16** acquiert une image en transmission de l'échantillon dans la gamme spectrale de la source lumineuse.

L'image *Ih* acquise par le capteur d'image **16** comprend des informations holographiques dans la mesure où elle résulte de l'interférence entre une onde *Fi* diffractée par les particules **11a-11f** et une onde de référence *Fn* ayant traversée l'échantillon sans avoir interagi avec lui. On comprend évidemment, comme décrit plus haut, que dans le cadre d'un capteur CMOS ou CCD, l'image numérique *Ih* acquise et mémorisée dans l'unité **20** est une image en intensité, l'information de phase étant donc ici codée en intensité, avec *Ih* selon la relation :

$$Ih = \begin{pmatrix} ih(1,1) & ... & ih(c,1) & ... & ih(C,1) \\ \vdots & \ddots & \vdots & ... & \vdots \\ ih(1,l) & ... & ih(c,l) & ... & ih(C,l) \\ \vdots & \ddots & \vdots & \ddots & \vdots \\ ih(1,L) & ... & ih(c,L) & ... & ih(C,L) \end{pmatrix}$$

Alternativement, il est possible de diviser le signal cohérent *Sn* issu de la source lumineuse **15** en deux composantes, par exemple au moyen d'une lame semi-transparente. La première composante sert alors d'onde de référence et la seconde composante est diffractée par l'échantillon **12**, l'image dans le plan image du système optique **23** résultant de l'interférence entre l'onde diffractée et l'onde de référence.

L'image en intensité *Ih* acquise par le capteur d'image **16** n'est pas focalisée sur la particule à observer et l'obtention d'une information focalisée sur la particule est obtenue numériquement par une unité de traitement **20** connectée au capteur d'image **16** pour recevoir les images acquises par ce dernier.

Par « défocalisée », on entend ici qu'il n'y a pas d'intersection entre le plan de mise au point et la particule objet de l'observation.

L'unité de traitement **20** peut correspondre à un ordinateur, un microcontrôleur, une tablette tactile ou un téléphone intelligent, ou de manière générale tout système informatique à base de processeur capable de recevoir des données, traiter ces données en mettant en oeuvre des instructions informatiques stockées dans une mémoire informatique, et de délivrer et/ou stocker dans une mémoire informatique le résultat du traitement. L'unité de traitement **20** peut être connectée de manière filaire ou non filaire avec le capteur d'image **16** ou au moyen d'une communication sans fil. L'unité peut être associée à un écran pour l'affichage des résultats intermédiaires ou finaux du procédé de l'invention.

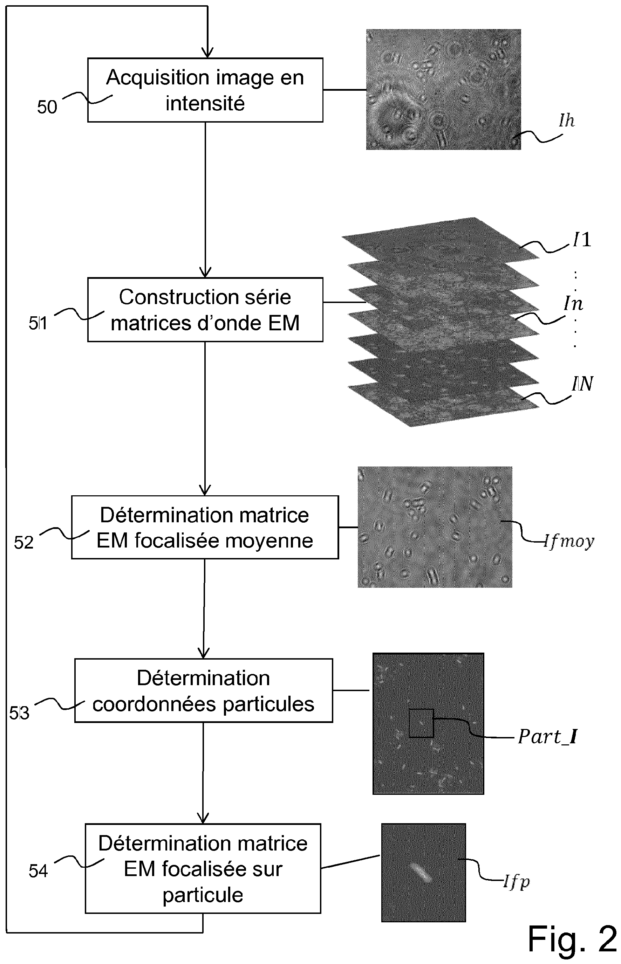

La Figure 2 illustre les modules de traitement informatique 51-53, intégrés dans l'unité de traitement **20** sous forme d'instructions informatiques mises en oeuvre par l'ordinateur, suite à l'acquisition **50** de l'image en intensité *Ih* par le capteur d'image **16.**

Un premier module **51** construit une série de matrices complexes *I1, ...,In, ...,IN,* nommées « matrices électromagnétiques » **EM,** modélisant à partir de l'image *Ih* le front d'onde lumineux propagé le long de l'axe optique pour une pluralité d'écarts par rapport au plan de mise au point du système optique **23,** et en particulier des écarts positionnés dans l'échantillon.

Une méthode de calcul de fronts d'onde par propagation numérique est expliqué dans l'article de Sang-Hyuk Lee et al. Intitulé « Holographie microscopy of holographically trapped three-dimensional structures » publié dans Optics Express, Vol. 15 ; N°4, 19 Février 2017, pp. 1505-1512.

Plus précisément, si l'on note $h_z(r)$ la fonction de propagation de Rayleigh-Sommerfeld, soit :

$$h_z(r) = -\frac{1}{2\pi}\frac{\partial}{\partial z}\frac{e^{ikR}}{R}$$

où :

- $z$ est la hauteur dite de « défocalisation », autrement dit l'écart par rapport au plan de mise au point,
- $r = (|r|, 0)$ est la position en coordonnées polaires dans le plan d'image, de coordonnée radiale $|r|$ et de coordonnées angulaire ,
- $R^2 = |r|^2 + z^2$ , et
- $k = 2\pi n/\lambda$ est un nombre d'onde relatif au milieu de propagation d'indice de réfraction $n$ à la longueur d'onde $\lambda$ de la source lumineuse.

A partir de cette relation, l'onde électromagnétique $a(r,z)$, d'amplitude $|a(r,z)|$ et de phase $\varphi(r,z)$, dans le plan d'ordonnée $z$ peut être exprimée sous la forme :

$$a(r,z) = |a(r,z)|\exp(i\varphi(r,z))$$

$$a(r,z) = \frac{1}{4\pi^2}\int_{-\infty}^{+\infty} B(q)H_{-z}(q)\exp(iqr)\,d^2q$$

où

- $b(r)$ est intensité mesurée, i.e. l'image *Ih* (l'intensité de l'onde de référence est ici supposée constante),
- $B(q)$ est la transformée de Fourier de $b(r)$,
- $H_{-z}(q)$ est la transformée de Fourier de $h_{-z}(r)$ , et
- $q$ est la variable duale de $r$ dans la transformée de Fourier.

Les équations ci-dessus définissent une formulation analytique de l'amplitude $a(r,z)$. Bien que ce modèle soit développé pour la propagation dans un milieu homogène (et donc sans modification du nombre d'onde, sans présence d'interface source de réflexion et/ou de déviation de l'onde, etc.), et par conséquent sans rapport avec l'échantillon et la chambre (qui comprennent de nombreuses interfaces et changement d'indices par exemple), les inventeurs ont noté qu'il permet de reconstruire des informations électromagnétiques riches en rapport avec les particules observées, comme cela sera décrit-après. Ainsi, avantageusement, l'unité de traitement **20** mémorise un seul nombre d'onde, commun pour tous les milieux en jeux, par exemple l'indice de l'air. En variante, l'unité **20** mémorise les indices de réfraction des différents milieux en jeu le long de l'axe optique et construit les matrices *I1 - IN* de proche en proche pour tenir compte les phénomènes aux interfaces.

Pour des bactéries, le pas d'échantillonnage en z est inférieur de préférence au dixième de l'épaisseur de la bactérie, par exemple inférieure à 0,1 µm, et de préférence inférieure à 0,03 µm.

On comprend ainsi que l'on peut construire une pile de matrices électromagnétiques *I1 - IN* pour des ordonnées $z_1, z_2, ..., z_n, z_N$ le long de l'axe optique, l'origine des ordonnées (z = 0) étant prise à la position axiale de mise au point, chaque matrice *In* étant définie par une amplitude complexe $a(r,z_n)$ selon les relations :

$$\begin{pmatrix} a(1,1)_{z_n} & ... & a(c,1)_{z_n} & ... & a(C,1)_{z_n} \\ \vdots & \ddots & \vdots & ... & \vdots \\ a(1,l)_{z_n} & ... & a(c,l)_{z_n} & ... & a(C,l)_{z_n} \\ \vdots & \ddots & \vdots & \ddots & \vdots \\ a(L,1)_{z_n} & ... & a(c,L)_{z_n} & ... & a(C,L)_{z_n} \end{pmatrix}$$

$$\forall (c,l) \in [1,C] \times [1,L] : a(c,l)_{z_n} = a\left( r\left((c,l), y(c,l)\right), z_n\right)$$

L'unité de traitement 20 calcule ensuite sur chaque matrice *In* une application surjective positive *AS* de l'espace complexe $\mathbb{C}^{C \times L}$ vers l'espace réel $\mathbb{R}^{C \times L}$ :

$$AS(In) = \begin{pmatrix} AS(a(1,1)_{z_n}) & ... & AS(a(c,1)_{z_n}) & ... & AS(a(C,1)_{z_n}) \\ \vdots & \ddots & \vdots & ... & \vdots \\ AS(a(1,l)_{z_n}) & ... & AS(a(c,l)_{z_n}) & ... & AS(a(C,l)_{z_n}) \\ \vdots & \ddots & \vdots & \ddots & \vdots \\ AS(a(L,1)_{z_n}) & ... & AS(a(c,L)_{z_n}) & ... & AS(a(C,L)_{z_n}) \end{pmatrix}$$

Par exemple, l'unité **20** calcule la norme hermitienne (ou son carrée) des composantes $a(c,l)_{z_n}$, la valeur absolue ou le carré de la partie imaginaire (notée $Im(a(c,l)_{z_n})$ ou de la partie réelle (notée $Re(a(c,l)_{z_n})$) des composantes $a(c,l)_{z_n}$, ou $Re^2(a(c,l)_{z_n}) + Im^2(a(c,l)_{z_n})$ ou $(Re^2(a(c,l)_{z_n}) + Im^2(a(c,l)_{z_n}))^{1/2}$.

Sans être lié par la théorie, les matrices *AS(I1) - AS(IN)* ne représentent pas nécessairement une intensité lumineuse, mais les inventeurs ont noté leur ressemblance avec des images en intensité obtenues sous éclairage non cohérent. Notamment, les particules sont représentées, comme dans une photographie, sous leur forme de particules.

Il est ainsi possible d'appliquer tout type de traitement d'image classique (segmentation, seuillage, détection de particule sur la base de leur morphologie, etc.), et même pour un opérateur d'identifier à l'oeil les particules (à la différence d'une image codant des interférences qui sont codées en intensité sous la forme de franges). Dans ce qui suit, pour alléger les notations, les matrices *AS(I1) - AS(IN)* sont notées *I1 - IN,* et la notation $a(c,l)_{z_n}$ correspondant à $AS(a(c,l)_{z_n})$.

Le procédé selon l'invention consiste ensuite à identifier des particules dans l'échantillon en fonction des matrices *I1 - IN,* et pour chaque particule identifiée, à déterminer une distance *z* de focalisation optimale, i.e. de mise au point, pour cette particule, puis à déterminer dans la matrice de la série *I1 - IN* correspondant à cette distance, un ensemble de pixels appartenant à cette particule.

Le second module **52** vise à déterminer une distance de focalisation moyenne *zfmoy* à partir de la série de matrices *I1- IN* et à sélectionner dans cette série la matrice, notée *Ifmoy,* dont la distance *z* est égale ou la plus proche de la distance *zfmoy.* En variante, l'unité **20** recalcule la matrice *Ifmoy* pour la distance *zfmoy.* Cette distance de focalisation moyenne *zfmoy* est celle qui correspond au mieux aux conditions idéales de mise au point sur l'ensemble des particules **11a-11f** au sens d'un critère de focalisation prédéterminé. Cette distance peut être déterminée par toutes les techniques connues de traitement du signal ou du domaine de la photographie, par exemple de l'autofocus. La matrice électromagnétique résultante *Ifmoy* est suffisamment « focalisée » pour des particules à différentes profondeurs puissent être détectées dans la matrice *Ifmoy.* Les particules détectées sont notamment celles comprises dans une profondeur d'échantillon égale à la profondeur de champ. Comme décrit précédemment, dans un mode de réalisation préféré, les particules sont disposées dans un volume d'épaisseur proche ou égale à cette profondeur de champ, de sorte que la totalité, ou la quasi-totalité, des particules de l'échantillon peuvent être détectées dans la matrice *Ifmoy.*

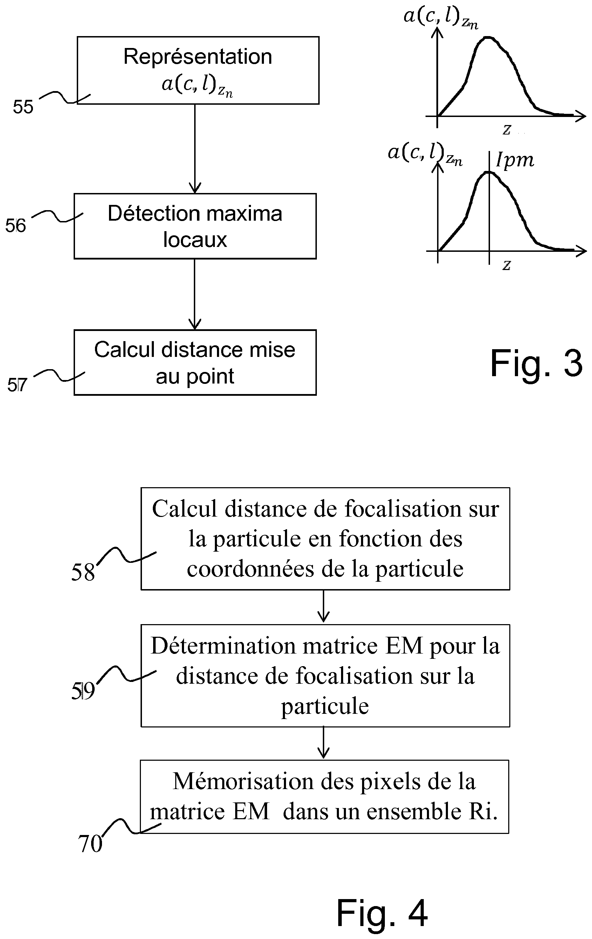

La figure 3 illustre un exemple de détermination de la distance de focalisation moyenne *zfmoy* en représentant, pour chaque coordonnée (*c, l*), la variation de $a(c,l)_{z_n}$ en fonction de la distance *z* dans la pile d'images *I1 - IN.* Lorsqu'une particule est située dans l'échantillon sur l'axe, parallèle à l'axe optique du système **23**, de coordonnée (c,l) dans le plan focal, on observe une variation de $a(c,l)_{zn}$. Par exemple, la représentation, en **55,** des variations $a(c,l)_{zn}$ est analogue à une fonction gaussienne en fonction de la distance **z.** Au contraire, lorsqu'aucune particule n'est présente sur cet axe, signifiant que seul le milieu de l'échantillon est présent, $a(c,l)_{zn}$ ne varie pas ou très peu. Il est ainsi possible de détecter, en **56,** un optimum **Ipm** pour chaque coordonnée (c, 1) représentant la distance **z** de mise au point particulière pour cette coordonnée. La distance de focalisation moyenne **zfmoy** peut donc être recherchée par l'unité **20** en calculant, en **57,** la moyenne des distances **z** obtenues en détectant l'optimum **Ipm** de chaque coordonnée (c,l). **Ifmoy** est donc la matrice de la série de matrices **I1 - IN** la plus proche de la distance **z** calculée. En variante, l'unité **20** sélectionne les **P** coordonnées, par exemple les 10000 coordonnées, présentant les plus grandes variations de leurs valeurs $a(c,l)_{zn}$ en fonction de **z** (e.g. présentant les plus grands écarts entre la valeur maximale et la valeur minimale), puis calcule la distance **zfmoy** sur ces **P** coordonnées, ce qui augmente la précision sur cette distance. En variante, les valeurs $a(c,l)_{zn}$ de ces **P** coordonnées sont moyennées et l'optimum de la courbe moyennée en fonction de **z** est calculé, la distance **z** de l'optimum étant la distance **zfmoy**

Bien que la distance de focalisation moyenne **zfmoy** illustre une mise au point générale de l'image, la mise au point de chaque particule **11a-11f** n'est pas optimale, notamment en raison des variations de profondeur des particules **11a-11f** entre elles. Pour améliorer la mise au point d'une particule **11a-11f** particulière, l'invention propose de déterminer une distance de focalisation optimale pour chaque particule et de déterminer une matrice focalisée spécifique à la particule. A cet effet, l'unité de traitement **20** comporte un module **53** d'identification des particules et un module **54** de détermination d'une distance de focalisation optimale pour chaque particule identifiée et de détermination des pixels de la particule pour cette distance.

Le module **53** d'identification des particules dans la matrice **Ifmoy** peut prendre plusieurs formes de segmentation d'image de l'état de la technique, tel qu'un balayage de cette matrice jusqu'à détecter les contours d'un élément fini. En variante, l'unité **20** applique un seuillage préalable à la matrice **Ifmoy,** la valeur seuil étant par exemple égale à $Moy(Ifmoy) + p \times E(Ifmoy)$, où $Moy(Ifmoy)$ est la moyenne des pixels de la matrice **Ifmoy,** $E(Ifmoy)$ est leur écart-type, et p un entier supérieur à 1, par exemple égal à 6 Les valeurs supérieures à ce seuil sont alors déterminées comme appartenant à des particules, et une segmentation d'image sur la matrice seuillée est mise en oeuvre. A l'issue de l'identification, il est ainsi obtenu *I* ensembles de coordonnées de pixel (c,l), notés **Part_1, ... , Part_i, ... Part_I.** Chaque ensemble, mémorisé dans l'unité **20,** répertorie les coordonnées des pixels de l'image **Ifmoy** appartenant à une même particule.

Le procédé détermine ensuite, en **54,** pour chaque ensemble de coordonnées **Part_i,** quelle distance **z** offre la meilleure focalisation pour la particule correspondante, puis détermine quelle matrice de la série **I1 - IN** correspond à cette distance (ou calcule un nouvelle image pour cette distance), et finalement mémorise dans un ensemble **Ri** les pixels de la matrice sélectionnée qui correspondent aux coordonnées listés dans l'ensemble **Part_i.**

La figure 4 illustre un exemple de détermination d'un ensemble **Ri.** Cette détermination débute, en **58,** par le calcul de la distance de focalisation optimale **zfopt_i,** par exemple de manière analogue au calcul de la distance de focalisation moyenne. Par exemple, pour chaque coordonnée (c,l) de l'ensemble **Part_i,** la distance correspondant à l'optimum de $a(c,l)_{zn}$ est calculée, puis la distance de focalisation optimale **zfopt_i** est choisie égale à la moyenne des distances **z** calculées. En variante, les valeurs $a(c,l)_{zn}$ de l'ensemble **Part_i** sont moyennées, et l'optimum de la courbe des valeurs moyennées en fonction de **z** est calculé, la distance **z** de l'optimum étant la distance **zfopt_i.** Ensuite, en **59,** la matrice de la série **I1 - IN** correspondant à la distance **zfopt_i.,** notée **I_i,** est sélectionnée. En **70,** les pixels de la matrice sélectionnée de coordonnées listées dans l'ensemble **Part_i** sont sélectionnés et mémorisés dans un ensemble **Ri.**

En outre, les modes de réalisation des figures 3 et 4 sont facilement combinables car ils utilisent les mêmes fonctions gaussiennes. Les traitements peuvent donc être effectués en parallèle de sorte à limiter le temps de calcul. Le temps de calcul peut également être réduit en sous-échantillonnant (e.g. : en **z** à une période de $0,1\,\mu m$) les matrices de la pile de matrice **I1 - IN** permettant de sélectionner l'ensemble **Ri.**

Pour chaque particule identifiée, une image **Ifp_i** est préférentiellement affichée sur un écran **22.** Cette image est par exemple une fenêtre rectangulaire de la matrice **I_i** comprenant les pixels **Ri.** Comme illustré aux figures, une bactérie en bâtonnet a effectivement la forme d'un bâtonnet dans la matrice focalisée **I_i.** Un praticien peut ainsi observer la forme de la particule **11a-11f** très rapidement sans effectuer de manipulation sur le système optique **23.** Pour améliorer l'analyse de la particule **11a-11f,** celle-ci peut être représentée au cours du temps par le biais de plusieurs acquisitions successives.

Pour ce faire, lorsque l'ensemble **Ri** est obtenu, il est stocké dans une mémoire **21** reliée à l'unité de traitement **20.** Après une durée prédéfinie, un nouvel ensemble **Ri** est recherché par le biais d'une nouvelle acquisition d'image **Ih,** le pas de temps étant inférieur ou égal à 5 minutes dans le cadre de bactéries, et de préférence inférieure ou égale à 1 minute. Les inventeurs ont noté au travers de leur invention que cette période permet de suivre les modifications phénotypiques ou morphologiques de tout type de bactérie. En effet, on observe, par la simple inspection visuelle d'une série temporelle d'images **Ifp_i** d'une bactérie, que certaines bactéries changent d'une minute à l'autre.

Le nouvel ensemble **Ri** doit correspondre à la même particule **11a-11f** que le premier ensemble **Ri.** Pour ce faire, le module **53** de détermination des ensembles *Part_1* - *Part_N* est réalisé en fonction de la position et/ou de la forme d'une particule **11a-11f** sélectionnée sur une matrice *Ifmoy* précédente. Les ensembles *Ri* focalisées *Ifp_i* sont tous stockés dans la mémoire **21** et l'écran **22** permet au praticien de lancer un flux d'images focalisées *Ifp_i* sur une particule **11a-11f** de sorte à analyser son comportement.

Le dispositif peut également réaliser l'analyse de plusieurs échantillons **12** simultanément, par exemple pour effectuer un antibiogramme d'une bactérie. Pour ce faire, l'unité de traitement **20** peut être reliée à plusieurs capteurs d'images **16** associés à plusieurs échantillons **12**. L'unité de traitement **20** effectue alors le traitement de plusieurs images *Ih* issues de plusieurs capteurs d'images **16** et l'écran **22** illustre les images focalisées *Ifp*.

Dans le mode de réalisation venant d'être décrit, la distance focale optimale *zfopt_i* correspond à une focalisation moyenne sur la particule, menant usuellement à une mise au point sur un plan médian de la particule. Ceci permet notamment de comparer les ensembles *Ri* entre plusieurs instants d'acquisition. En variante, une focalisation est recherchée pour chaque pixel (e.g. calcul de la distance correspondant à l'optimal de $a(c,l)_{zn}$ en fonction de *z,* recherche de la matrice électromagnétique à cette distance, mémorisation dans l'ensemble *Ri* du pixel de cette matrice de coordonnée (c,l)), ce qui permet de construire une visualisation 3D de la particule. Par contre, la comparaison entre deux reconstructions successives de la particule est plus difficile.

L'acquisition selon l'invention étant réalisée sans focus, on peut acquérir à une fréquence très élevée, et si besoin faire le traitement après avoir acquis.

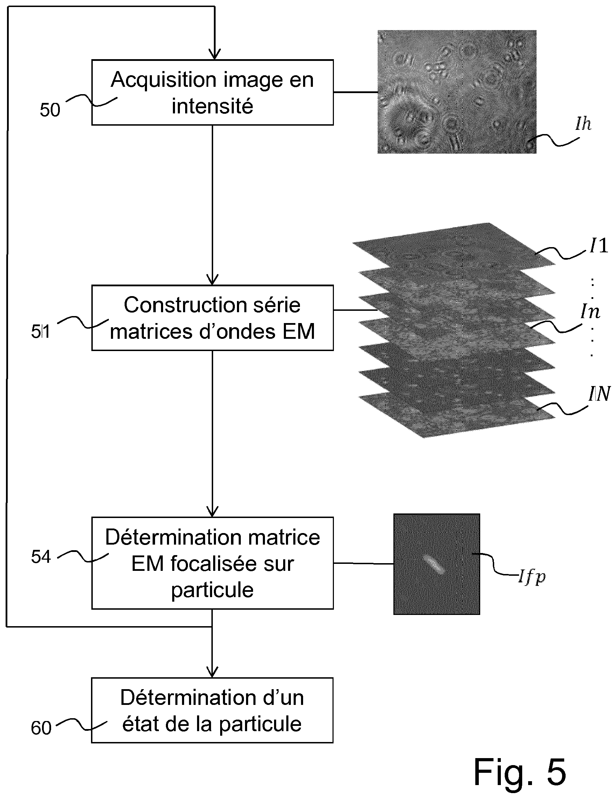

La figure 5 illustre un second mode de réalisation de l'invention consistant à détecter la division d'un microorganisme en fonction du temps. Dans ce mode de réalisation, l'unité de traitement **20** comporte un module de détermination **60** d'un état de la particule **11a-11f** en fonction de l'évolution temporelle de l'ensemble de pixels *Ri* associé au microorganisme, e.g. l'état phénotypique ou morphologique d'une bactérie.

Dans le mode de réalisation de la figure 5, la détermination **54** de l'ensemble de pixels *Ri* peut être effectuée par une simple recherche de focalisation numérique à partir de la pile d'images *I1* - **IN** contrairement au mode de réalisation de la figure 2 dans lequel une double recherche de focalisation est effectuée. Les pixels de l'ensemble *Ri* sont alors ceux de la matrice *Ifmoy.* En variante, la détermination **54** de l'ensemble de pixels *Ri* peut être réalisée avec un processus analogue à celui de la figure 2.

Le module de détermination **60** d'un état de la particule **11a-11f** peut mettre en oeuvre des indicateurs et des traitements différents. Par exemple, l'état de la particule **11a-11f** peut être analysé selon l'état de son cycle de division, ses caractéristiques morphologiques, ses caractéristiques physiologiques ou une combinaison de plusieurs indicateurs.

De préférence, les indicateurs sont définis en fonction de la nature de la particule **11a-11f** dont l'état est recherché.

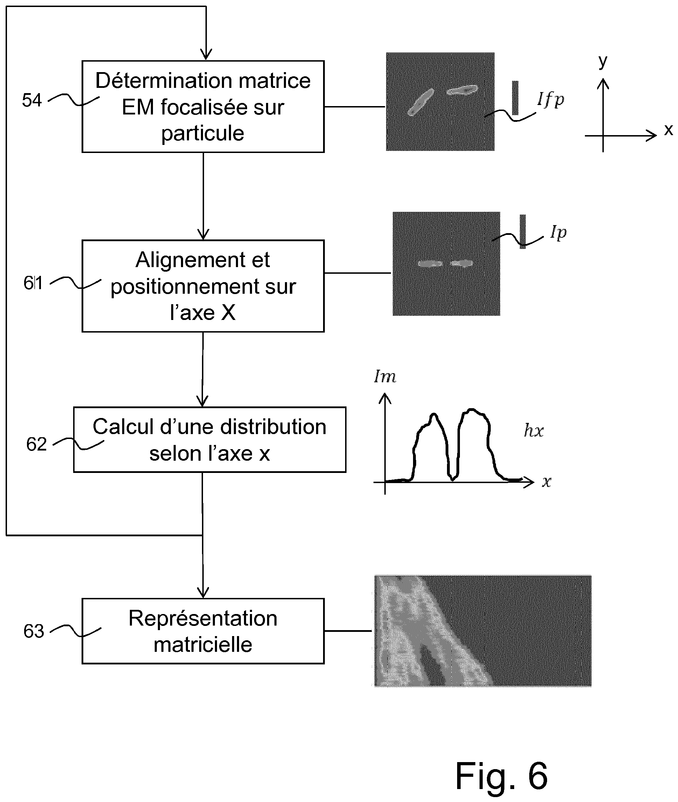

Pour une particule **11a-11f** analogue à une bactérie en présence d'un antibiotique, le mode de réalisation de la figure 6 est particulièrement efficace. Dans ce mode de réalisation, des images d'intensité *Ih* sont acquises périodiquement, avantageusement toute les minutes pour les raisons décrites plus haut, et pour chaque image *Ih* acquise, l'ensemble *Ri* associé à la bactérie est déterminé

Une rotation **61** est appliquée à l'ensemble *Ri* de sorte à aligner et positionner la particule **11a-11f** sur un axe prédéfini constant dans le temps, par exemple en alignant à gauche la particule sur l'axe *x* des abscisses (i.e l'axe correspondant aux lignes des images acquises). L'ensemble *Ri* aligné est ensuite projeté sur l'axe d'alignement, par exemple en réalisant la moyenne des pixels sur chaque ordonnée *y* (i.e. l'axe des colonnes des images acquises). La distribution *Ip* ainsi obtenue est représentée 62 sous la forme d'une distribution de la somme *Im* par colonne en fonction de la position de l'ordonné *x* des pixels, et est par exemple mémorisée dans l'unité **20** sous la forme d'une vecteur ligne.

Les distributions sont ensuite représentées au cours du temps en concaténant dans une matrice les distributions *Ip.* En notant *Ip_q* la distribution obtenue à partir de la qième image *Ih* acquise, et *Q* le dernier instant d'acquisition, la matrice, notée *Dist_Q,* s'écrit ainsi sous la forme :

$$Dist\_Q = \begin{pmatrix} Ip\_1 \\ \vdots \\ Ip\_q \\ \vdots \\ Ip\_Q \end{pmatrix}$$

La représentation matricielle ainsi obtenue est particulièrement efficace pour déterminer l'état de la particule **11a-11f.** Par exemple, cette représentation matricielle permet d'observer l'allongement d'une particule ou la présence d'au moins une division cellulaire.

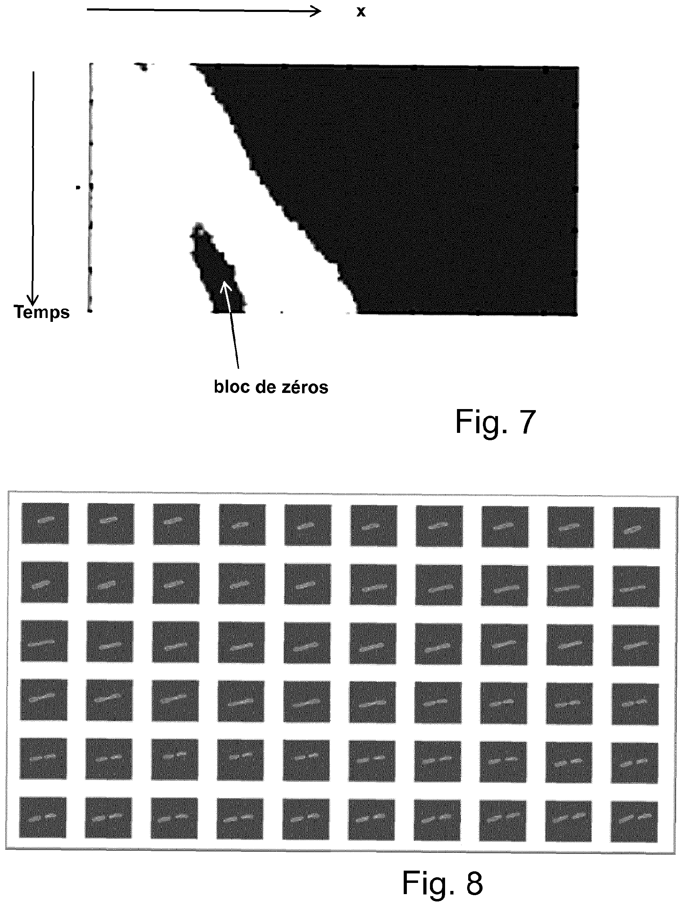

Un simple seuillage sur la forme de la représentation matricielle permet alors de définir si un antibiotique a agi efficacement ou non sur une bactérie. Notamment, comme illustré à la figure 7, on voit apparaitre dans la matrice seuillée, composée de zéro et de un, un bloc de zéros encadré par deux blocs de 1, l'apparition du bloc de zéros marquant l'instant de début de division, et s'accroitre. L'identification par l'unité de traitement **20** de l'apparition d'un tel bloc permet ainsi d'identifier d'une manière simple la division de la bactérie. En variante, d'autres analyses peuvent être effectuées en modélisant la vitesse de déplacement d'une particule **11a-11f** entre les images holographique *Ih* captée par le capteur d'image **16** ou en analysant les caractéristiques structurelles d'une particule **11a-11f** par correspondance avec des particules de forme connue.

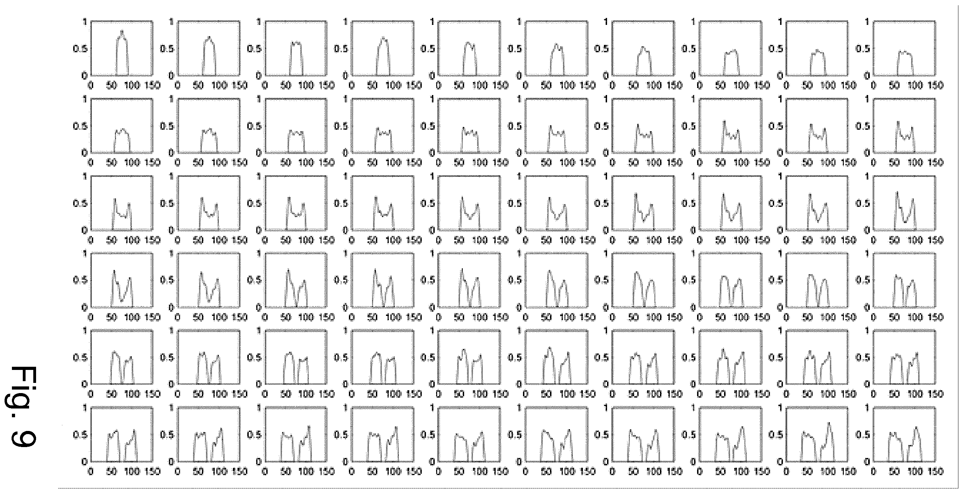

L'invention permet ainsi d'obtenir une image précise d'une particule **11a-11f** présente dans un échantillon **12**. Typiquement, l'invention a permis d'obtenir une imagette représentant une bactérie avec un nombre de pixels compris entre 100 et 400 pixels. Il s'ensuit qu'il est possible visuellement ou numériquement de visualiser, et ce sans marquage (e.g. fluorescent), le contenu d'un microorganisme. Notamment, on peut observer la répartition du matériel intracellulaire dans une bactérie. Sans être lié à la théorie, les inventeurs ont pu observer l'apparition de deux pôles dans une bactérie avant sa division, ce qui pourrait correspondre à la méiose d'une bactérie, comme illustré sur la figure 8 qui représente en fonction du temps l'évolution d'une bactérie. L'unité de traitement **20** met ainsi en oeuvre au cours du temps un traitement de l'ensemble *Ri* d'une bactérie, ou de la distribution *Ip_q,* afin de déterminer l'apparition de deux zones de valeur analogue. Une division de la bactérie est ainsi prédite bien avant que cette division n'ait lieu. La figure 9 illustre les distributions *Ip_q* de la bactérie illustrée à la figure 8. Comme on peut le constater à cette figure, lorsque la bactérie se prépare à la division, il apparait deux pôles dans la distribution, bien avant la division elle-même qui se traduit par deux portions distinctes de la distribution. L'unité de traitement **20** est par exemple configurée pour détecter l'apparition de deux pôles dans la distribution et prédire en fonction de cette détection la division de la bactérie, et par conséquent sa susceptibilité à l'antibiotique présent dans l'échantillon.

En outre, l'invention permet également d'obtenir l'évolution de la particule **11a-11f** au cours du temps afin de visualiser son comportement, par exemple sa vitesse de déplacement ou son processus de division cellulaire.

Notamment grâce à la résolution obtenue par l'invention (plusieurs centaines de pixels par bactérie pour système conçu par les inventeurs), il est possible d'observer l'influence d'un antibiotique sur la morphologie et le contenu intracellulaire des bactéries. En outre, la cadence élevée d'acquisition de l'invention permet traquer de manière très fine temporellement les variations de la bactérie (e.g. croissance, division, réaction à un milieu de culture, à un milieu avec antibiotique, etc.)

Il a été décrit un objectif dans l'air et à distance de l'échantillon. D'autres types d'agencement sont possibles, par exemple un objectif microscopique immergé dans une cuve de liquide recevant l'échantillon et/ou pouvant se déplacer par rapport à l'échantillon.

**Revendications**

**1.** Dispositif de détermination de l'état d'au moins une particule par acquisition (10) au cours du temps d'une pluralité de particules (11a-11f) présentes dans un échantillon (12), ledit dispositif d'acquisition (10) comportant :

- une source lumineuse (15), spatialement cohérente ou pseudo-cohérente, orientée sur une première face (13) dudit échantillon (12) ;
- un système optique (23) ayant un axe optique et réalisant la conjugaison entre un plan de mise au point et un plan focal, orienté sur une seconde face (14) dudit échantillon (12) opposé à ladite première face (13), et placé par rapport à l'échantillon de sorte que les particules ne sont pas dans le plan de mis au point ;
- un capteur d'image (16), placé dans le plan focal du système optique et configuré pour acquérir une image en intensité (Ih) formée par l'interférence entre ladite source lumineuse (15) et ledit échantillon (12) ; et
- une unité de traitement informatique (20) comportant :

    o un module (51) de construction numérique d'une série de matrices électromagnétiques (*I1- IN*) modélisant, à partir de l'image acquise *(Ih),* l'onde électromagnétique dans des plans parallèles au plan de mise au point et compris dans l'échantillon pour une pluralité d'écarts par rapport audit plan,
    o un module (52) de détermination d'une première matrice électromagnétique *(Ifmoy)* à une distance de mise au point moyenne sur les particules (11a-11f) à partir de la série de matrices électromagnétiques (*I1 - IN)* ;
    o un module (53) d'identification d'au moins une des particules (11a-11f) dans la première matrice électromagnétique *(Ifmoy)* et de mémorisation des coordonnées de ladite particule ;
    o un module (54) de détermination d'une seconde matrice électromagnétique (*Ifp*) à une distance de mise au point sur une particule identifié (11a-11f) à partir des composantes de la série de matrices électroma-gnétique (*I1 - IN*) ayant les coordonnées mémorisées, et
    o un module (60) de détermination d'un état de la particule (11a-11f) en fonction de l'évolution temporelle des coordonnées mémorisées.

2. Dispositif d'acquisition selon la revendication 1, *dans lequel* ledit capteur d'image (16) est configuré pour acquérir un flux d'images (*Ih*), et dans lequel l'unité de traitement est configurée pour suivre une particule dans le flux de premières matrices électromagnétiques *(Ifmoy)* .

3. Dispositif d'acquisition selon la revendication 2, *dans lequel* les particules sont des microorganismes, et dans lequel la durée entre deux images acquises (*Ih*) issues dudit capteur d'image (16) est inférieure ou égale à 1 minute.

4. Dispositif d'acquisition selon l'une des revendications 1 à 3, *dans lequel* ladite série de matrices électromagnétiques (*I1 - IN*) est obtenue par un modèle de propagation numérique d'une lumière à travers ledit échantillon (12), les matrices électromagnétiques (*I1 - IN*) variant en modulant une distance (z) d'un axe optique dudit modèle de propagation.

5. Dispositif d'acquisition selon la revendication 4, *dans lequel* l'unité de traitement comprend un module de transformation des matrices électromagnétiques issues du modèle de propagation par une application surjective de l'espace complexe dans l'espace réel.

6. Dispositif d'acquisition selon la revendication 4 ou 5, *dans lequel* la première matrice électromagnétique (*Ifmoy*) est obtenue en représentant, à chaque coordonnée, les composantes à ladite coordonnée des matrices électromagnétiques (*I1 - IN*) en fonction de ladite distance (z) dudit axe optique, et en recherchant une moyenne des maxima de toutes les représentations.

7. Dispositif d'acquisition selon la revendication 6, *dans lequel* la seconde image électromagnétique est obtenue en représentant, à chaque coordonnée de la particule identifiée, les composantes à ladite coordonnée des matrices électromagnétiques (*I1 - IN*) en fonction de ladite distance (z) dudit axe optique, et en recherchant une moyenne des maxima de toutes les représentations.

8. Dispositif d'acquisition selon l'une des revendications précédentes, *dans lequel* les matrices de ladite série de matrices électromagnétiques (*I1 - IN*) utilisées pour déterminer ladite première matrices électromagnétiques (*Ifmoy*) et/ou pour déterminer ladite seconde matrice électromagnétique sont sous-échantillonnées selon ladite distance.

9. Dispositif d'acquisition selon l'une des revendications précédentes, *dans lequel* ledit dispositif comporte plusieurs unités d'acquisition, chaque unité d'acquisition comportant un capteur d'image et des moyens de focalisation spécifiques, ledit dispositif étant configuré pour représenter une image d'une particule de chaque échantillon.

10. Procédé d'analyse de l'état d'au moins une particule (11a-11f) présente dans un échantillon (12), ledit procédé d'analyse comportant les étapes suivantes :

    - émission d'une source lumineuse (15), spatialement cohérente ou pseudo-cohérente, orientée sur une première face (13) dudit échantillon (12) ;
    - acquisition (50) à l'aide d'un capteur d'image d'une image en intensité (*Ih*), ledit capteur étant placé dans le plan focal d'un système optique (23) ayant un axe optique et réalisant la conjugaison entre un plan de mise au point et le plan focal, orienté sur une seconde face (14) dudit échantillon (12) opposé à ladite première face (13), et placé par rapport à l'échantillon de sorte que la particule n'est pas dans le plan de mis au point, l'image (*Ih*) étant formée par interférence entre ladite source lumineuse (15) et ledit échantillon (12) ;
    - construction numérique d'une série de matrices électromagnétiques (*I1 - IN*) modélisant, à partir de l'image acquise *(Ih),* l'onde électromagnétique dans des plans parallèles au plan de mise au point et compris dans l'échantillon pour une pluralité d'écarts par rapport audit plan ;
    - obtention (54) d'une première matrice électromagnétique *(Ifmoy)* à une distance de mise au point moyenne sur les particules (11a-11f) à partir de la série de matrices électromagnétique (*I1- IN*) ;
    - identification d'au moins une particule (11a-11f) dans la première matrice électromagnétique ($I_{fmoy}$) et mémorisation des coordonnées de ladite particule ;
    - obtention d'une seconde matrice électromagnétique (*Ifp*) à une distance de mise au point sur une particule identifiée à partir des composantes de la série de matrices électromagnétique (*I1 - IN*) ayant les coordonnées mémorisée ; et
    - les étapes d'acquisition d'une image *(Ih)* jusqu'à l'étape d'obtention de la matrice électromagnétique (*Ifp*) à la distance de mise au point sur la particule étant réalisées au cours du temps pour plusieurs images, détermination d'un état (60) de ladite particule (11a-11f) en fonction d'une évolution temporelle de la matrice électromagnétique (*Ifp*) à la distance de mise au point sur la particule (11a-11f)

11. Procédé d'analyse selon la revendication 10, *dans lequel* ladite étape de détermination d'un état de ladite particule (11a-1 1f) comporte les étapes suivantes :

- rotation (61) de la matrice électromagnétique (*Ifp*) à la distance de mise au point sur la particule de manière à aligner la particule sur un axe prédéfini ; et
- calcul (62) d'une distribution de la matrice alignée selon ledit axe prédéfini.

12. Procédé d'analyse selon la revendication 11, *dans lequel* ladite étape de détermination d'un état de ladite particule (11a-11f) comporte les étapes suivantes :

- rotation (61), pour chaque image acquise, de la matrice électromagnétique (*Ifp*) à la distance de mise au point sur la particule de manière à aligner la particule sur un axe prédéfini ;
- calcul (62), pour chaque image acquise, d'une distribution de la matrice alignée selon ledit axe prédéfini ; et
- représentation (63) matricielle des distributions associées à chaque image en fonction du temps.

13. Procédé d'analyse selon l'une des revendications 10 à 12, *dans lequel* ladite étape de détermination (60) d'un état de ladite particule (11a-11f) est réalisée en fonction d'un état de division de ladite particule (11a-11f)

14. Procédé d'analyse selon l'une des revendications 10 à 13, *dans lequel* ladite étape de détermination (60) d'un état de ladite particule (11a-11f) est réalisée en fonction d'une caractéristique morphologique de ladite particule (11a-11f), par exemple la longueur.

15. Procédé d'analyse selon l'une des revendications 10 à 14, *dans lequel* ladite étape de détermination (60) d'un état de ladite particule (11a-11f) est réalisée en fonction d'une caractéristique physiologique de ladite particule (11a-11f), par exemple le nombre de noyau.

16. Procédé d'analyse selon l'une des revendications 10 à 15, *dans lequel* ladite particule (11a-11f) correspond à une bactérie et ledit échantillon (12) intègre un antibiotique.

**Patentansprüche**

1. Vorrichtung zur Bestimmung des Zustandes mindestens eines Partikels durch Erfassung (10) über die Zeit einer Vielzahl von Partikeln (11a-11f), die in einer Probe (12) vorhanden sind, diese Erfassungsvorrichtung (10) umfasst dabei:

- eine Leuchtquelle (15), räumlich kohärent oder pseudo-kohärent, ausgerichtet hin zu einer ersten Seite (13) dieser Probe (12);
- ein optisches System (23), mit einer optischen Achse, das die Konjugation zwischen einer Scharfstellebene und einer Brennebene realisiert, ausgerichtet auf eine zweite Seite (14) dieser Probe (12) gegenüber der erwähnten ersten Seite (13), und bezogen auf die Probe so ausgerichtet, dass sich die Partikel nicht innerhalb der Scharfstellebene befinden;
- ein Bildsensor (16), angeordnet in der Brennebene des optischen Systems und dazu konfiguriert ein Lichtstärkebild *(Ih.)* zu erfassen, gebildet aus der Interferenz zwischen dieser Leuchtquelle (15) und dieser Probe (12); sowie
- eine Computerbearbeitungseinheit (20), die enthält:

  o ein digitales Konstruktionsmodul (51) einer Reihe elektromagnetischer Matrizen *(I1 - IN)* die, ausgehend von dem erfassten Bild *(Ih),* die elektromagnetische Welle in Ebenen parallel zur Scharfstellebene modellieren und in der Probe für eine Vielzahl von Abweichungen zu dieser Ebene enthalten sind,
  o ein Modul zur Bestimmung (52) einer ersten elektromagnetischen Matrix *(Ifmoy)* in einer Entfernung des durchschnittlichen Scharfstellens der Partikel (11 a-11f) ausgehend von einer Reihe elektromagnetischer Matrizen *(/I - IN*;
  o ein Identifikationsmodul (53) mindestens eines der Partikel (11a-11f) in der ersten elektromagnetischen Matrix *(Ifmoy)* und zur Speicherung der Koordinaten dieses Partikels;

o ein Modul zur Bestimmung (54) einer zweiten elektromagnetischen Matrix *(Ifp)* in einem Schaftstellungsabstand für ein identifiziertes Partikel (11a-11f), ausgehend von Komponenten der Reihe elektromagnetischer Matrizen *(I1 - IN)* mit gespeicherten Koordinaten, und
o ein Modul zur Bestimmung (60) eines Zustandes des Partikels (11a-11f) in Abhängigkeit von der zeitlichen Entwicklung der gespeicherten Koordinaten.

2. Erfassungsvorrichtung nach Anspruch 1, *in der* der erwähnte Bildsensor (16) dazu konfiguriert ist, einen Bilderfluss *(Ih),* aufzunehmen und in der die Bearbeitungseinheit konfiguriert ist, um einem Partikel im Fluss der ersten elektromagnetischen Matrizen *(Ifmoy).* zu folgen.

3. Erfassungsvorrichtung nach Anspruch 2, *in der* die Partikel Mikroorganismen sind, und in der die Dauer zwischen zwei erfassten Bildern *(Ih)* aus diesem Bildsensor (16) kleiner oder gleich 1 Minute ist.

4. Erfassungsvorrichtung nach einem der Ansprüche 1 bis 3, *in der* diese Reihe elektromagnetischer Matrizen (71 - *IN)* erhalten wird über ein digitales Ausbreitungsmodell eines Lichts durch diese Probe (12), die elektromagnetischen Matrizen (71 - *IN)* variieren dabei, wobei sie einen Abstand (z) einer optischen Achse von diesem Ausbreitungsmodell modulieren.

5. Erfassungsvorrichtung nach Anspruch 4, *in der* die Bearbeitungseinheit ein Modul zur Transformation der elektromagnetischen Matrizen aus dem Ausbreitungsmodell durch eine surjektive Anwendung des komplexen Raums im realen Raum umfasst.

6. Erfassungsvorrichtung nach Anspruch 4 oder 5, *in der* die erste elektromagnetische Matrix *(Ifmoy)* erhalten wird durch Darstellung in jeder Koordinate, der Komponenten dieser Koordinate der elektromagnetischen Matrizen (71 - *IN)* in Abhängigkeit von diesem Abstand (z) dieser optischen Achse und in der Suche nach einem Durchschnitt der Maxima aller Darstellungen.

7. Erfassungsvorrichtung nach Anspruch 6, *in der* das zweite elektromagnetische Bild erhalten wird durch Darstellung in jeder Koordinate, des identifizierten Partikels der Komponenten dieser Koordinate der elektromagnetischen Matrizen (71 - *IN)* in Abhängigkeit von diesem Abstand (z) dieser optischen Achse und in der Suche nach einem Durchschnitt der Maxima aller Darstellungen.

8. Erfassungsvorrichtung nach einem der vorangehenden Ansprüche, *in der* die Matrizen dieser Reihe elektromagnetischer Matrizen *(I1 - IN),* verwendet zur Bestimmung der erwähnten ersten elektromagnetischen Matrix *(Ifmoy)* und/ oder zur Bestimmung der erwähnten zweiten elektromagnetischen Matrix entsprechend diesem Abstand mit ungenügender Abtastrate erfasst werden.

9. Erfassungsvorrichtung nach einem der vorangehenden Ansprüche, *in der* diese Vorrichtung mehrere Erfassungseinheiten umfasst, jede Erfassungseinheit enthält dabei einen Bildsensor und spezifische Fokussiermittel, diese Vorrichtung ist dazu konfiguriert, ein Bild eines Partikels jeder Probe zu repräsentieren.

10. Verfahren zur Analyse des Zustandes mindestens eines in einer Probe (12) vorhandenen Partikels (11a-11f), dieses Analyseverfahren enthält dabei die folgenden Schritte:

   - Emission einer Leuchtquelle (15), räumlich kohärent oder pseudo-kohärent, ausgerichtet hin zu einer ersten Seite (13) dieser Probe (12);
   - Erfassung (50) eines Lichtstärkebildes *(Ih)* mit Hilfe eines Bildsensors, dieser Sensor ist dabei in der Brennebene eines optischen Systems (23) angeordnet, das eine optische Achse hat und das die Konjugation zwischen einer Scharfstellebene und einer Brennebene realisiert, ausgerichtet auf eine zweite Seite (14) dieser Probe (12) gegenüber der erwähnten ersten Seite (13), und bezogen auf die Probe so ausgerichtet, dass sich das Partikel nicht innerhalb der Scharfstellebene *(Ih)* befindet; das Bild wird dabei durch Interferenz zwischen der erwähnten Leuchtquelle (15) und der erwähnten Probe (12) gebildet;
   - digitale Konstruktion einer Reihe elektromagnetischer Matrizen *(I1 - IN)*, zur Modellierung, ausgehend von dem erfassten Bild *(Ih),* der elektromagnetischen Welle in Ebenen parallel zur Scharfstellebene und in der Probe für eine Vielzahl von Abweichungen zu dieser Ebene enthalten;
   - Erhalt (54) einer ersten elektromagnetischen Matrix *(Ifmoy)* in einer Entfernung des durchschnittlichen Scharfstellens der Partikel (11a-11f) ausgehend von einer Reihe elektromagnetischer Matrizen *(I1 - IN)*;
   - Identifikation (53) mindestens eines der Partikel (11a-11f) in der ersten elektromagnetischen Matrix $(I_{fmoy})$ und Speicherung der Koordinaten dieses Partikels;

- alt (54) einer zweiten elektromagnetischen Matrix (Ifp) in einem Scharfstellungsabstand für ein identifiziertes Partikel (11a-11f), ausgehend von Komponenten der Reihe elektromagnetischer Matrizen (I1 - IN), die die gespeicherten Koordinaten haben; und

- die Schritte zur Erfassung eines Bildes (Ih) bis zum Schritt des Erhalts der elektromagnetischen Matrix (Ifp) in einem Scharfstellungsabstand für das Partikel, werden dabei im Laufe der Zeit für mehrere Bilder realisiert, Bestimmung eines Zustandes (60) dieses Partikels (11a-11f) in Abhängigkeit von einer zeitlichen Entwicklung der elektromagnetischen Matrix (Ifp) im Schaftstellungsabstand für das Partikel (11a-11f)

11. Analyseverfahren nach Anspruch 10, *in dem* der Schritt zur Bestimmung eines Zustandes dieses Partikels (11a-11f) die folgenden Schritte enthält:

- Rotation (61) der elektromagnetischen Matrix (Ifp) auf den Scharfstellungsabstand für das Partikel, um so das Partikel an einer vorher festgelegten Achse auszurichten; und
- Berechnung (62) einer Verteilung der entlang der vorher festgelegten Achse ausgerichteten Matrix; und

12. Analyseverfahren nach Anspruch 11, *in dem* dieser Schritt zur Bestimmung eines Zustandes dieses Partikel (11a-11f) die folgenden Schritte enthält:

- Rotation (61), für jedes erfasste Bild, der elektromagnetischen Matrix (Ifp) auf den Scharfstellungsabstand für das Partikel, um so das Partikel an einer vorher festgelegten Achse auszurichten; und
- Berechnung (62), für jedes erfasste Bild, einer Verteilung der entlang der vorher festgelegten Achse ausgerichteten Matrix;
- Matrix-Darstellung (63) der mit jedem Bild verknüpften Verteilungen in Abhängigkeit von der Zeit.

13. Analyseverfahren nach einem der Ansprüche 10 bis 12, *in dem* der Schritt zur Bestimmung (60) eines Zustandes dieses Partikels (11a-11f) in Funktion eines Teilungszustandes dieses Partikels (11a-11f). realisiert wird.

14. Analyseverfahren nach einem der Ansprüche 10 bis 13 *in dem* dieser Schritt zur Bestimmung (60) eines Zustandes dieses Partikels (11a-11f) in Funktion einer morphologischen Eigenschaft dieses Partikels (11a-11f) erfolgt, zum Beispiel der Länge.

15. Analyseverfahren nach einem der Ansprüche 10 bis 14, *in dem* dieser Schritt zur Bestimmung (60) eines Zustandes dieses Partikels (11a-11f) in Funktion einer physiologischen Eigenschaft dieses Partikels (11a-11f) realisiert wird, zum Beispiel die Kernzahl.

16. Analyseverfahren nach einem der Ansprüche 10 bis 15, *in dem* dieses Partikel (11a-11f) einer Bakterie entspricht und diese Probe (12) ein Antibiotikum enthält.

**Claims**

1. A device of determination of the state of at least one particle by acquisition (10) over time of a plurality of particles (11a-11f) present in a sample (12), said acquisition device (10) comprising:

- a spatially coherent or pseudo-coherent light source (15) directed towards a first surface (13) of said sample (12);
- an optical system (23) having an optical axis and achieving the conjugation between a plane of focus and a focal plane, directed towards a second surface (14) of said sample (12) opposite to said first surface (13), and placed relative to the sample so that the particles are not in the plane of focus;
- an image sensor (16), placed in the focal plane of the optical system and configured to acquire an intensity image (Ih) formed by the interference between said light source (15) and said sample (12); and
- a computational processing unit (20) comprising:

o a unit (51) of digital construction of a series of electromagnetic matrices (I 1 - IN) modeling, from the acquired image (Ih), the electromagnetic wave in planes parallel to the plane of focus and comprised in the sample for a plurality of offsets relative to said plane,

o a unit (52) for determining a first electromagnetic matrix (Ifmoy) at an average distance of focus on the particles (11a-11f) from the series of electromagnetic matrices (I1- IN);

o a unit (53) for identifying at least one of the particles (11a-11f) in the first electromagnetic matrix *(Ifmoy)* and for storing the coordinates of said particle;

o a unit (54) for determining a second electromagnetic matrix *(Ifp)* at a distance of focus on a particle (11a-11f) identified from the components of the series of electromagnetic matrices *(I1- IN)* having the stored coordinates ; and

o a unit (60) for determining a state of particle (11a-11f) as a function of the time variation of the stored coordinates.

2. The acquisition device of claim 1, ***wherein*** said image sensor (16) is configured to acquire an image flow (*Ih*), and wherein the processing unit is configured to track a particle in the flow of first electromagnetic matrices *(Ifmoy).*

3. The acquisition device of claim 2, ***wherein*** the particles are microorganisms and ***wherein*** the duration between two acquired images (*Ih*) originating from said image sensor (16) is shorter than or equal to 1 minute.

4. The device of any of claims 1 to 3, ***wherein*** said series of electromagnetic matrices (I 1 - *IN)* is obtained by a model of digital propagation of light through said sample (12), the electromagnetic matrices *(I1- IN)* varying by modulating a distance (z) to an optical axis of said propagation model.

5. The acquisition device of claim 4, ***wherein*** the processing unit comprises a unit for transforming the electromagnetic matrices originating from the propagation model by a surjective application from the complex space into the real space.

6. The acquisition device of claim 4 or 5, ***wherein*** the first electromagnetic matrix ( *Ifmoy)* is obtained by representing, for each coordinate, the components at said coordinate of the electromagnetic matrices *(I1- IN)* according to said distance (z) to said optical axis, and by searching for an average of the maximum values of all representations.

7. The acquisition device of claim 6, ***wherein*** the second electromagnetic image is obtained by representing, for each coordinate of the identified particle, the components at said coordinate of the electromagnetic matrices *(I1- IN)* according to said distance (z) to said optical axis, and by searching for an average of the maximum values of all representations.

8. The acquisition device of any of the foregoing claims, ***wherein*** the matrices of said series of electromagnetic matrices *(I1- IN)* used to determine said first electromagnetic matrices *(Ifmoy)* and/or to determine said second electromagnetic matrix are sub-sampled according to said distance.

9. The acquisition device of any of the foregoing claims, ***wherein*** said device comprises a plurality of acquisition units, each acquisition unit comprising an image sensor and specific focusing means, said device being configured to represent an image of a particle of each sample.

10. A method of analysis of the state of at least one particle (11a-11f) present in a sample (12), said analysis method comprising the steps of:

- emitting a spatially coherent or pseudo-coherent light source (15) directed towards a first surface (13) of said sample (12);

- acquiring (50) by means of an image sensor an intensity image (*Ih*), said sensor being placed in the focal plane of an optical system (23) having an optical axis and performing the conjugation between a plane of focus and the focal plane, directed towards a second surface (14) of said sample (12) opposite to said first surface (13), and placed relative to the sample so that the particle is not in the plane of focus, the image (Ih) being formed by interference between said light source (15) and said sample (12);

- digitally constructing a series of electromagnetic matrices *(I1- IN)* modeling, from the acquired image *(Ih),* the electromagnetic wave in planes parallel to the plane of focus and comprised in the sample for a plurality of offsets with respect to said plane;

- obtaining (54) a first electromagnetic matrix *(Ifmoy)* at an average distance of focus on the particles (11a-11f) from the series of electromagnetic matrices (I 1 - *IN);*

- identifying of at least one particle (11a-11f) in the first electromagnetic matrix ( *Ifmoy)* and storing the coordinates of said particle;

- obtaining a second electromagnetic matrix (*Ifp*) at a distance of focus on said identified particle from the coordinates of the series of electromagnetic matrices ( *I1- IN)* having the stored coordinates;

- the steps of acquisition of an image ( *Ih* ) until the step of obtaining the electromagnetic matrix (*Ifp*) at the distance of focus on the particle being carried out over time for a plurality of images, the step (60) of determining a state of said particle (11a-11f) as a function of a time variation of the electromagnetic matrices (*Ifp*) at the distance of focus on the particle (11a-11f).

11. The analysis method of claim 10, ***wherein*** said step of determining a state of said particle (11a-11f) comprises the steps of:

- rotating (61) the electromagnetic matrix (*Ifp*) at the distance of focus on the particle to align the particle with a predefined axis; and
- calculating (62) a distribution of the matrix aligned along said predefined axis.

12. The analysis method of claim 11, ***wherein*** said step of determining a state of said particle (11a-11f) comprises the steps of:

- rotating (61) for each acquired image, the electromagnetic matrix at the distance of focus (*Ifp*) on the particle to align the particle with a predefined axis;
- calculating (62), for each acquired image, a distribution of the matrix aligned along said predefined axis; and
- creating a matrix representation (63) of the distributions associated with each image over time.

13. The analysis method of any of claims 10 to 12, ***wherein*** said step of determining (60) a state of said particle (11a-11f) is carried out according to a division state of said particle (11a-11t).

14. The analysis method of any of claims 10 to 13, ***wherein*** said step of determining (60) a state of said particle (11a-11f) is carried out according to a morphological characteristic of said particle (11a-1 1f), for example, the length.

15. The analysis method of any of claims 10 to 14, ***wherein*** said step of determining (60) a state of said particle (11a-11f) is carried out according to a physiological characteristic of said particle (11a-11f), for example, the nuclear number.

16. The analysis method of any of claims 10 to 15, ***wherein*** said particle (11a-11f) corresponds to a bacterium and said sample (12) integrates an antibiotic.

Fig. 1

Fig. 2

$a(c,l)_{z_n}$

$z$

$a(c,l)_{z_n}$ $Ipm$

$z$

## Fig. 3

Représentation
$a(c,l)_{z_n}$

55

Détection maxima
locaux

56

Calcul distance mise
au point

57

Calcul distance de focalisation sur
la particule en fonction des
coordonnées de la particule

58

Détermination matrice EM pour la
distance de focalisation sur la
particule

59

Mémorisation des pixels de la
matrice EM dans un ensemble Ri.

70

## Fig. 4

Fig. 5

Fig. 6

bloc de zéros

Fig. 7

Fig. 8

Fig. 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2603601 A

**Littérature non-brevet citée dans la description**

- **MYUNG K.KIM.** Principles and techniques of digital holographie microscopy. *SPIE Reviews,* Janvier 2010, vol. 1 (1
- **N. WU et al.** Three-dimensional identification of microorganisms using a digital holographie microscope. *Computational and Mathematical Methods in Medicine,* vol. 2013
- **AHMED EL MALLAHI.** Automated threedimensional détection and classification of living organisms using digital holography microscopy with partial spatial cohérent source: application to monitoring of drinking water resources. *Applied Optics,* Janvier 2013, vol. 52 (1
- **SANG-HYUK LEE et al.** Holographie microscopy of holographically trapped three-dimensional structures. *Optics Express,* 19 Février 2017, vol. 15 (4), 1505-1512